# EUROPÄISCHE PATENTSCHRIFT

(11) **EP 0 611 774 B1**
(45) Veröffentlichungstag und Bekanntmachung des Hinweises auf die Patenterteilung: **26.05.1999**
(21) Anmeldenummer: 94101784.0
(22) Anmeldetag: 07.02.1994
(51) Int. Cl.: C07K 1/02

(54) **Racemisierungsfreie Herstellung von Amiden und Peptiden in Gegenwart katalytischer Mengen einer N-Hydroxy-Verbindung**
Production of amides and peptides without racemization in the presence of catalytic amounts of a N-hydroxy-compound
Production des amides et des peptides sans racemisation en présence de quantités catalytiques d'un composé N-hydroxylé

(30) Priorität: 19.02.1993 CH 518/93
(43) Veröffentlichungstag der Anmeldung: 24.08.1994
(73) Patentinhaber: F. HOFFMANN-LA ROCHE AG, 4002 Basel (CH)
(72) Erfinder: Hohler, Markus, CH-4313 Möhlin (CH); Vogt, Peter, CH-4147 Aesch (CH)
(74) Vertreter: Kellenberger, Marcus, Dr.

(56) Entgegenhaltungen:
- CHEM. BER. Bd. 103 , 1970 Seiten 788 - 798 KÖNIG, W. & GEIGER, R. 'Eine neue Methode zur Synthese von Peptiden ...'
- ROCZNIKI CHEMII, ANN.SOC.CHIM.POLONORUM Bd. 51, Nr. 5 , 1977 Seiten 939 - 949 PRZYBYLSKI, J. ET AL. 'Influence of additives on suppression of racemation in peptide synthesis ...'
- BODANSZKY, M. 'Principles of peptide synthesis, 2nd ed.' , SPRINGER-VERLAG Chapter 1.1: "Racemisation" (pages 170-185)

## Beschreibung

Die vorliegende Erfindung betrifft ein neues Verfahren zur Herstellung von Amiden und Peptiden durch Umsetzen einer Carbonsäure mit einem primären oder sekundären Amin in Gegenwart eines Carbodiimides und einer N-Hydroxyverbindung, wobei die N-Hydroxyverbindung in katalytischer Menge vorliegt und worin N-Hydroxysuccinimid, 1-Hydroxybenzotriazol, 3-Hydroxy-4-oxo-3,4-dihydro-1,2,3-benzotriazin, 1-Hydroxy-2-oxoindolin, 3-Hydroxy-4-oxo-3,4-dihydrochinazolin oder 1-Hydroxy-2(1H)-pyridon, insbesondere N-Hydroxysuccinimid oder 1-Hydroxy-2(1H)-pyridon, als N-Hydroxyverbindung verwendet wird.

Als "Carbonsäuren" im Rahmen der vorliegenden Erfindung kommen unsubstituierte und substituierte aliphatische, aromatische, aromatischaliphatische, heteroaromatische oder heteroaromatisch-aliphatische Carbonsäuren oder natürliche N-acylierte α-Aminosäuren mit der L-Konfiguration, Homologe solcher Aminosäuren, z.B. worin die Aminosäureseitenkette um eine oder zwei Methylengruppen verlängert oder verkürzt ist und/oder eine Methylgruppe durch Wasserstoff ersetzt ist, substituierte aromatische N-acylierte α-Aminosäuren, z.B. substituiertes Phenylalanin oder Phenylglycin, worin der Substituent Alkyl, z.B. Methyl, Halogen, z.B. Fluor, Chlor, Brom oder Jod, Hydroxy, Alkoxy, z.B. Methoxy, Alkanoyloxy, z.B. Acetoxy, Amino, Alkylamino, z.B. Methylamino, Dialkylamino, z.B. Dimethylamino, Alkanoylamino, z.B. Acetylamino oder Pivaloylamino, Alkoxycarbonylamino, z.B. t-Butoxycarbonylamino, Arylmethoxycarbonylamino, z.B. Benzyloxycarbonylamino, 9-Fluorenylmethoxycarbonyl und/oder Nitro sein kann und ein- oder mehrfach vorkommt, benzanneliertes Phenylalanin oder Phenylglycin, wie α-Naphthylalanin, oder hydriertes Phenylalanin oder Phenylglycin, wie Cyclohexylalanin oder Cyclohexylglycin, eine 5- oder 6-gliedrige cyclische benzannelierte N-acylierte α-Aminosäure, z.B. Indolin-2-carbonsäure oder 1,2,3,4-Tetrahydroisochinolin-3-carbonsäure, eine natürliche oder homologe N-acylierte α-Aminosäure, in der eine Carboxygruppe in der Seitenkette in veresterter oder amidierter Form vorliegt, z.B. als Alkylestergruppe, wie Methoxycarbonyl oder t-Butoxycarbonyl, oder als Carbamoylgruppe, Alkylcarbamoylgruppe, wie Methylcarbamoyl, oder als Dialkylcarbamoylgruppe, wie Dimethylcarbamoyl, in der eine Aminogruppe der Seitenkette in acylierter Form vorliegt, z.B. als Alkanoylaminogruppe, wie Acetylamino oder Pivaloylamino, als Alkoxycarbonylaminogruppe, wie t-Butoxycarbonylamino, oder als Arylmethoxycarbonylaminogruppe, wie Benzyloxycarbonylamino, oder in der eine Hydroxygruppe der Seitenkette in verätherter oder veresterter Form vorliegt, z.B. als Alkoxygruppe, wie Methoxy, als Arylalkoxygruppe, wie Benzyloxy, oder als nieder Alkanoyloxygruppe, wie Acetoxy, oder Epimere solcher Aminosäuren, d.h. mit der unnatürlichen D-Konfiguration in Frage. Geeignete N-Acylgruppen sind Alkanoyl, wie Acetyl oder Pivaloyl, Alkoxycarbonyl, wie t-Butoxycarbonyl, Arylalkoxycarbonyl, wie Benzyloxycarbonyl, oder der Acylrest einer der nachstehend genannten aromatisch-aliphatischen oder heteroaromatischen Carbonsäuren, wie (S)-α-[(t-Butylsulfonyl)methyl]-β-phenylpropionyl, (S)-α-[[[1-(Morpholinocarbonyl)-1-methylethyl]sulfonyl]methyl]-β-phenylpropionyl oder 2-Chinolylcarbonyl, oder einer der oben erwähnten Aminosäuren oder eines Dipeptids bestehend aus zwei der oben erwähnten Aminosäuren.

Beispiele von geeigneten unsubstituierten und substituierten aliphatischen, aromatischen und aromatisch-aliphatischen Carbonsäuren sind Propionsäure, Isobuttersäure, (R)-Milchsäure, (S)-Milchsäure, 2-Phthalimidoxyisobuttersäure, Benzoesäure, 3,4-Dihydroxybenzoesäure, Salicylsäure, 1-Naphthoesäure, 2-Naphthoesäure, Phenylessigsäure, p-Hydroxyphenylessigsäure, (S)-α-[(t-Butylsulfonyl)methyl]hydrozimtsäure und (S)-α-[[[1-(Morpholinocarbonyl)-1-methylethyl]sulfonyl]methyl]hydrozimtsäure. Als Beispiele geeigneter heteroaromatischer oder heteroaromatisch-aliphatischer Carbonsäuren können genannt werden 2-Pyridincarbonsäure, 3-Pyridincarbonsäure, 4-Pyridincarbonsäure, 5-Chlor-2-pyridincarbonsäure, 2-Pyrimidincarbonsäure, 4-Pyrimidincarbonsäure, 2-Chinolincarbonsäure, 3-Chinolincarbonsäure, 2-Pyridylessigsäure, 3-Indolylessigsäure, 3-(3-Indolyl)propionsäure, Isochinolin-1-carbonsäure und (4-Imidazolyl)essigsäure.

Beispiele geeigneter, oben beschriebener Aminosäuren sind Glycin, Alanin, Valin, Norvalin, Leucin, Isoleucin, Norleucin, Serin, Homoserin, Threonin, Methionin, Cystein, Prolin, Trans-3- und Trans-4-hydroxyprolin, Phenylalanin, Tyrosin, 4-Nitrophenylalanin, 4-Aminophenylalanin, 4-Chlorphenylalanin, β-Phenylserin, Phenylglycin, α-Naphthylalanin, Cyclohexylalanin, Cyclohexylglycin, Tryptophan, Indolin-2-carbonsäure, 1,2,3,4-Tetrahydroisochinolin-3-carbonsäure, Asparaginsäure, Asparagin, Aminomalonsäure, Aminomalonsäuremonoamid, Glutaminsäure, Glutaminsäuremono-t-butylester, Glutamin, N-Dimethylglutamin, Histidin, Arginin, Lysin, N-t-Butoxycarbonyllysin, δ-Hydroxylysin, Ornithin, N-Pivaloylornithin, α,γ-Diaminobuttersäure oder α,β-Diaminopropionsäure und dergleichen.

Als "primäre oder sekundäre Amine" im Rahmen der vorliegenden Erfindung kommen insbesondere Aminosäuren in Frage, wie sie oben beschrieben wurden, welche jedoch mindestens eine nicht-acylierte Aminogruppe aufweisen und deren Säuregruppe beispielsweise durch Veresterung geschützt ist. Im weiteren kommen auch Alkylamine, Dialkylamine, Arylalkylamine oder gegebenenfalls durch ein Sauerstoff-, Schwefel- oder gegebenenfalls Alkyl-, Phenylalkyl-, Alkanoyl- oder Alkanoyloxy-substituiertes Stickstoffatom unterbrochene C₃₋₆-Alkylen-disubstituierte Amine, welche noch weitere, unter den Reaktionsbedingungen inerte Reste enthalten können, sowie gegebenenfalls durch Alkyl oder Acyl substituiertes Hydrazin in Frage.

Als Beispiele solcher Amine können die Methylester der weiter oben aufgezählten Aminosäuren, wie Histidinmethylester, sowie t-Butyl (2-aminoethyl)carbamat, (1S,2R,3S)-3-Amino-4-cydohexyl-1-cyclopropyl-butan-1,2-diol, 2-(3(S)-Amino-2(R)-hydroxy-4-phenylbutyl)-N-t-butyl-decahydro-(4aS,8aS)-isochinolin-3(S)-carboxamid, t-Butyl (4-piperidinyloxy)acetat, Hydrazin, Methylhydrazin und 3,4-Dihydroxybenzoesäurehydrazid genannt werden.

Beispiele geeigneter Carbodiimide sind Dicyclohexylcarbodiimid (DCC), Diisopropylcarbodiimid (DIC), N-Ethyl-N'-(3-dimethylaminopropyl)carbodiimid-hydrochlorid (EDC), N-Cyclohexyl-N'-(β-[N-methylmorpholino]ethyl)carbodiimid-p-toluolsulfonat und dergleichen.

Als N-Hydroxyverbindungen kommen N-Hydroxysuccinimid, 1-Hydroxybenzotriazol, 3-Hydroxy-4-oxo-3,4-dihydro-1,2,3-benzotriazin, 1-Hydroxy-2-oxoindolin, 3-Hydroxy-4-oxo-3,4-dihydrochinazolin, 1-Hydroxy-2(1H)-pyridon in Frage, also Verbindungen mit einer anderen Struktur als die in der Patentanmeldung WO 94/07910 beschriebenen Verbindungen.

Die Herstellung von Amiden und Peptiden durch Umsetzen einer Carbonsäure mit einem primären oder sekundären Amin in Gegenwart von Dicyclohexylcarbodiimid und einer N-Hydroxyverbindung ist aus der Literatur bekannt. So wird in der Zeitschrift für Naturforschung (B), 426 (1966) beschrieben, dass die Peptidbildung aus N-Acylpeptiden, die eine optisch aktive Aminosäure carboxylendständig enthalten, praktisch racemisierungsfrei verläuft, wenn zur Peptidbildung DCC unter Zusatz von 2 Moläquivalenten N-Hydroxysuccinimid in Tetrahydrofuran oder Dimethylformamid bei -20°C verwendet wird. Aus Chemische Berichte 103 (1970), Seiten 788-798, 2024-2033 sowie 2034-2040 ist bekannt, dass der eben beschriebene Effekt auch beobachtet werden konnte, wenn anstelle von 2 Moläquivalenten N-Hydroxysuccinimid 1-2 Moläquivalente verschiedener 1-Hydroxybenzotriazole, 1-Hydroxy-2-oxoindoline, 3-Hydroxy-4-oxo-3,4-dihydrochinazoline und 3-Hydroxy-4-oxo-3,4-dihydro-1,2,3-benzotriazine eingesetzt wurden. Gemäss Journal of Organic Chemistry 37, 288 (1972) wurde 1 Moläquivalent N-Hydroxysuccinimid erfolgreich zur Unterdrückung der Bildung von N-Acylharnstoffen (Nebenprodukte bei der DCC-Aktivierung) bei der Umsetzung von 1-(9-Adenyl)-2,3-O-isopropyliden-β-D-ribofuranuronsäure mit Benzylestern verschiedener Aminosäuren und Peptiden. In Chemische Berichte 106, 3626 (1973) ist beschrieben, dass die Aminolyse von mit elektronenziehenden Resten substituierten Phenylestern durch N-Hydroxyverbindungen, die etwa die Acidität der Essigsäure besitzen, in polaren Lösungsmitteln stark beschleunigt wird, wobei sich insbesondere die Verwendung von 1 Moläquivalent 1-Hydroxybenzotriazol, 1-Hydroxy-2(1H)-pyridon und 3-Hydroxy-4-oxo-3,4-dihydrochinazolin bewährt habe. Gemäss Journal of the American Chemical Society 94, 3590 (1972) wird N-Hydroxysuccinimid zur Herstellung von aktivierten N-Hydroxysuccinimidestern in einer Menge von 1,1 Moläquivalenten eingesetzt.

Im Rahmen der vorliegenden Erfindung wurde nun festgestellt, dass sich Amide und Peptide mit erheblicher Reduktion der Abfallprodukte der Reaktion, jedoch unter Beibehaltung der hohen Ausbeuten und praktisch ohne Racemisierung herstellen lassen, wenn man die Umsetzung einer Carbonsäure mit einem primären oder sekundären Amin und einem Carbodiimid in Gegenwart von nur katalytischen Mengen einer N-Hydroxyverbindung durchführt.

Das erfindungsgemässe Verfahren ist demnach dadurch gekennzeichnet, dass man eine Carbonsäure mit einem primären oder sekundären Amin in Gegenwart einer äquimolaren Menge eines Carbodiimids und einer katalytischen Menge einer N-Hydroxyverbindung umsetzt.

Die Umsetzung erfolgt in an sich bekannter Weise, zweckmässig in einem unter den Reaktionsbedingungen inerten organischen Lösungsmittel oder Lösungsmittelgemisch bei einer Temperatur zwischen etwa 0°C und 50°C, vorzugsweise bei etwa Raumtemperatur. Als Lösungsmittel kommen insbesondere Ethylacetat, Aceton, Acetonitril, Dimethylformamid, Methylenchlorid, Tetrahydrofuran und dergleichen in Frage. Dabei kann durchaus auch die Methode der Festphasensynthese verwendet werden, falls das primäre oder sekundäre Amin eine Carboxylgruppe enthält. Daraus geht hervor, dass sich diese Methode besonders zur Herstellung von Peptiden eignet, wobei dann die Amidkopplung mehrmals hintereinander durchgeführt wird. Geeignete Ausgangsstoffe sind die weiter oben beschriebenen α-Aminosäuren, die an der Aminogruppe durch t-Butoxycarbonyl oder 9-Fluorenylmethoxycarbonyl geschützt sind. Als Träger eignen sich Polystyrol- oder Polyamid-Harze, wie p-Benzyloxybenzylalkohol-polystyrolharz, p-Hydroxymethyl-phenoxy-polystryrolharz, 4-(2',4'-Dimethoxyphenyl-hydroxymethyl)phenoxy-polystryrolharz, Dimethylacrylamid-polyamidharz, Glycylacrylamid-polyamidharz, Kiegelgur/Polyamidharze und dergleichen. Das gewünschte Peptid kann dann leicht vom Trägerharz abgespalten werden, beispielsweise mittels Trifluoressigsäure und dergleichen.

In den nachfolgenden Beispielen, welche die vorliegende Erfindung illustrieren, deren Umfang jedoch in keiner Weise einschränken sollen, sind sämtliche Temperaturen in Celsiusgraden angegeben.

### Beispiel 1

17,9 g (74 mMol) Histidinmethylester-dihydrochlorid, 70 ml Acetonitril und 20.6 ml (148 mMol) Triethylamin wurden 2 Stunden bei 20° gerührt. 20 g (70 mMol) (S)-α-[(t-Butylsulfonyl)methyl]hydrorimtsäure, 0.78 g (7 mMol) 1-Hydroxy-2(1H)-pyridon und 140 ml Ethylacetat wurden anschliessend zugegeben. Nach 15 Minuten gab man eine Lösung aus 15.2 g (74 mMol) Dicyclohexylcarbodiimid in 90 ml Ethylacetat innerhalb von 30 Minuten zu. Nach 18 Stunden Rühren bei 20° war der Umsatz vollständig. Der Festkörper (Dicyclohexylharnstoff) wurde abgenutscht, und das Filtrat mit wässeriger Natriumbicarbonatlösung und Wasser gewaschen. 32.3 g (S)-α-[(t-Butylsulfonyl)methyl]hydrocinnamamido]imidazolpropionsäuremethylester wurden als weisser Schaum erhalten (HPLC-Gehalt 94%; gehaltskorrigierte Ausbeute: 98%).

### Beispiel 2

2.5 g (10.5 mMol) Histidinmethylester-dihydrochlorid, 10 ml Acetonitril und 2.9 ml (148 mMol) Triethylamin wurden 2 Stunden bei 20° gerührt. 3.8 g (10 mMol) (S)-α-[[[1-(Morpholinocarbonyl)-1-methylethyl)sulfonyl]methyl]hydrozintsäure, 0.11 g (1 mMol) 1-Hydroxy-2(1H)-pyridon und 20 ml Ethylacetat wurden anschliessend zugegeben. Nach 15 Minuten gab man eine Lösung aus 2.2 g (10.5 mMol) Dicyclohexylcarbodiimid in 15 ml Ethylacetat innerhalb von 30 Minuten zu. Nach 18 Stunden Rühren bei 20° war der Umsatz vollständig. Der Festkörper (Dicyclohexylharnstoff) wurde abgenutscht, und das Filtrat mit wässeriger Natriumbicarbonatlösung und Wasser gewaschen. 5.4 g N-[(S)-α-[[[1-Methyl-1-(morpholinocarbonyl)ethyl]sulfonyl]methyl]hydrocinnamoyl]-L-histidinmethylester wurden erhalten.

### Beispiel 3

89 g (170 mMol) (S)-1-(t-Butoxycarbonyl)-α-[(S)-α-[(t-butylsulfonyl)methyl)hydrocinnamamido]imidazol-4-propionsäure, 1 g (8,5 mMol) N-Hydroxysuccinimid, 35.5 g (155 mMol) (1S,2R,3S)-3-Amino-4-cyclohexyl-1-cyclopropyl-butan-1,2-diol und 800 ml Ethylacetat wurden bei 20° gerührt. Innerhalb von 10 Minuten wurde eine Lösung aus 37 g (178 mMol) Dicyclohexylcarbodiimid in 110 ml Ethylacetat zugegeben. Nach 17 Stunden Rühren bei 20° war der Umsatz vollständig. Der Festkörper (Dicyclohexylharnstoff) wurde abfiltriert, und das Filtrat unter Rühren zuerst mit 68 ml entionisiertem Wasser und dann mit 1100 ml Hexan versetzt. Nach einer Stunde bei 0° wurde das Kristallisat abgenutscht und in Methanol bei 20° verrührt. Die Suspension wurde auf -15° abgekühlt, und das Produkt abfiltriert. Man erhielt 98.8 g (86%) 4-[(S)-2-[(S)-2-t-Butansulfonylmethyl-3-phenylpropionylamino]-2-[(1S,2R,3S)-1-cyclohexylmethyl-3-cyclopropyl-2,3-dihydroxypropylcarbamoyl]ethyl]-1H-imidazol-1-carbonsäure-t-butylester.

### Beispiel 4

11.7 g (44 mMol) N-(Benzyloxycarbonyl)-L-asparagin, 16.0 g (40 mMol) 2-[3(S)-Amino-2(R)-hydroxy-4-phenylbutyl]-N-t-butyl-decahydro-(4aS,8aS)isochinolin-3(S)-carboxamid und 0.46 g (4 mMol) N-Hydroxysuccinimid wurden bei 20° in 160 ml Tetrahydrofuran und 80 ml Ethylacetat suspendiert. Eine Lösung aus 9.1 g (44 mMol) Dicyclohexylcarbodiimid und 80 ml Ethylacetat wurde innerhalb von 15 Minuten zugetropft, und das Reaktionsgemisch bei 20° weitergerührt. Nach 18 Stunden war der Umsatz vollständig. Der Festkörper (Dicyclohexylharnstoff) wurde abgenutscht. Das Filtrat wurde vom Tetrahydrofuran befreit, und das Produkt aus Ethylacetat/Hexan kristallisiert. Die Ausbeute an cis-2-[3(S)-((N-(Benzyloxycarbonyl)-L-asparaginyl)amino]-2(R)-hydroxy-4-phenylbutyl]-N-t-butyldecahydro-(4aS,8aS)-isochinolin-3(S)-carboxamid betrug 23.0 g (88%).

### Beispiel 5

10.2 g (30 mMol) N-(Benzyloxycarbonyl)-L-tyrosin-dihydrat, 6.48 g (30 mMol) t-Butyl (4-piperidinyloxy)acetat sowie 0.17 g (1.5 mMol) N-Hydroxysuccinimid wurden in 200 ml Ethylacetat unter Rühren und unter Argon gelöst. Zur gelblichen Lösung wurden 6.5 g (31.5 mMol) Dicyclohexylcarbodiimid, gelöst in 33 ml Ethylacetat, innerhalb von 19 Minuten getropft. Langsam bildete sich eine Suspension, welche 22 Stunden bei 22° gerührt wurde. Der ausgefällte Dicyclohexylharnstoff wurde abfiltriert, und das Filtrat mit 1N Salzsäure, Wasser und Kochsalzlösung extrahiert. Die organische Phase wurde über Natriumsulfat getrocknet und nach Entfernen des Trockungsmittels eingedampft. 13.1 g t-Butyl [[1-[N-[(benzyloy)carbonyl]-L-tyrosyl]-4-piperidinyl]oxy]acetat wurden als weisser, harter Schaum erhalten, dessen Mikroanalyse derjenigen authentischen Materials entsprach.

### Beispiel 6

11.14 g (30 mMol) N-(Benzyloxycarbonyl)-O-(1,1-dimethlethyl)-L-tyrosin wurden unter Erwärmen auf 40° in 65 ml Ethylacetat gelöst. Die Lösung wurde bei 20° mit 0.1 g (0.9 mMol) N-Hydroxysuccinimid versetzt. Anschliessend wurde eine Lösung aus 7.5 g (36 mMol) Dicyclohexylcarbodiimid in 40 ml Ethylacetat innerhalb von 25 Minuten bei 22° zugetropft. Während der Zugabe entstand eine weisse Suspension, die 30 Minuten nachgerührt wurde. Zur Suspension wurde dann eine Lösung aus 7.8 g (36 mMol) t-Butyl (4-piperidinyloxy)acetat in 100 ml Ethylacetat innerhalb von 30 Minuten getropft. Langsam bildete sich eine Suspension, welche 3 Stunden bei 22° gerührt wurde. Der ausgefällte Dicyclohexylharnstoff wurde abfiltriert, und das Filtrat mit 2N Salzsäure, Wasser und halbgesättigter Natriumbicarbonatlösung gewaschen. Die organische Phase wurde über Natriumsulfat getrocknet und nach Entfernen des Trocknungsmittels eingedampft. Der Rückstand war ein viskoses Oel und wog 18.5 g. Das Oel wurde mit einem Gemisch aus 10 ml Ethylacetat und 60 ml Hexan verdünnt und auf 50° erwärmt. Der unlösliche Festkörper wurde abgenutscht, und das Filtrat mit 20 ml Hexan versetzt und bei 30° mit wenig kristallinem Produkt angeimpft. Das Gemisch wurde innerhalb einer Stunde auf 0° abgekühlt und 30 Minuten bei dieser Temperatur gerührt. Die Kristalle wurden dann abgenutscht und auf der Nutsche mit 20 ml Hexan gewaschen. Man erhielt auf diese Weise 14.5 g (85%) Benzyl [(S)-p-t-butoxy-α-[[4-[(t-butoxycarbonyl)methoxy]piperidino]carbonyl]phenethyl]carbamat. Dessen Gehalt betrug nach HPLC 96%.

### Beispiel 7

125.9 g (312 mMol) 2-(3(S)-Amino-2(R)-hydroxy-4-phenylbutyl)-N-t-butyldecahydro-(4aS,8aS)-isochinolin-3(S)-carboxamid, 94.6 g (328 mMol) N-(2-Chinolylcarbonyl)-L-asparagin und 3.5 g (31 mMol) 1-Hydroxy-2(1H)-pyridon wurden vorgelegt und mit einem Gemisch aus 75 ml Tetrahydrofuran und 1925 ml Ethylacetat versetzt. Unter Rühren wurde anschliessend bei 25° eine Lösung aus 70.9 g (344 mMol) Dicyclohexylcarbodiimid in 500 ml Ethylacetat innerhalb von 30 Minuten zugetropft. Man liess das Reaktionsgemisch 10 Stunden weiter rühren. Danach wurden 50 ml ionenarmes Wasser zugegeben, und die Suspension auf 2-3° abgekühlt und eine Stunde bei dieser Temperatur gerührt. Der Dicyclohexylharnstoff wurde dann abfiltriert und mit 2 Portionen Ethylacetat zu je 500 ml auf der Nutsche gewaschen. Die Filtrate wurden vereinigt und bei 50° langsam mit einer Lösung aus 32.5 g (338 mMol) Methansulfonsäure und 250 ml Ethylacetat versetzt. Anschliessend wurde die Suspension 14 Stunden bei 20° gerührt. Die Kristalle wurden dann abgenutscht, mit total 600 ml Ethylacetat gewaschen und anschliessend 24 Stunden bei 45°/2000 Pa getrocknet. Man erhielt 229.5 g (95%) N-t-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid-methansulfonat mit einem HPLC-Gehalt von 97.1%.

### Beispiel 8

H-Val-Gln-Ala-Ala-Ile-Asp-Tyr-Ile-Asn-Gly-OH wurde mittels Festphasen-Synthese unter Verwendung von Basen-labilen N-Fluorenylmethoxycarbonyl-Aminosäuren (Fmoc-Aminosäuren), t-Butyl-Seitenketten-Schutzfunktionen und p-Benzyloxybenzylalkohol-polystyrolharz, wie in Atherton und Sheppard in "The Peptides; Analysis, Synthesis, Biology", Vol. 9 beschrieben, hergestellt (S. Udenfriend and J. Meienhofer, Eds.; Academic Press, New York [1987]).
Die Synthese wurde mit 4 g Fmoc-Gly-OCH₂C₆H₄O-CH₂C₆H₄-Harz mit einer Beladung von 0.5 mMol Fmoc-Gly pro Gramm Harz, d.h. mit 2 mMol Substrat, gestartet. Verwendet wurde ein halbautomatischer "Peptide Synthesizer" SP 640 von der Labortec AG, CH-4416 Bubendorf. Folgende Gruppen wurden als Schutzfunktionen der Seitenketten verwendet: für Tyrosin der t-Butyläther und für Asparaginsäure der t-Butylester. Für den jeweiligen Kopplungsschritt wurden 2.5 Moläquivalente der nächstfolgenden Fmoc-Aminosäure, bezogen auf die am Harz gekoppelte Aminokomponente, eingesetzt. Als Kopplungsreagens diente 1 Moläquivalent DCC in Gegenwart von 0.1 Moläquivalent 1-Hydroxy-2(1H)-pyridon, bezogen auf die Fmoc-Aminosäure. Bei schwierigen Kopplungsschritten, wie der Kopplung von Valin an Glutamin, wurde ein 1:1-Lösungsmittelgemisch von Dimethylformamid (DMF) und 1,3-Dimethyl-2-imidazolidinon verwendet.

| Programm für einen Zyklus: | | |
|---|---|---|
| Operation | Lösungsmittel/Reagens | Wiederholungen/Dauer |
| Waschen | DMF | 2 x 1 Min. |
| Entschützen | 20% Piperidin in DMF | 1 x 3 Min. |
| Entschützen | 20% Piperidin in DMF | 1 x 10 Min. |
| Waschen | DMF | 4 x 0.5 Min. |
| Waschen | Isopropanol | 2 x 0.5 Min. |
| Stop | Ninhydrin-Test (muss positiv sein) | |
| Waschen | DMF | 2 x 1 Min. |
| Stop | Zugabe der nächsten Fmoc-Aminosäure sowie von 0.1 Mol-äquivalent 1-Hydroxy-2(1H)-pyridon in DMF | |
| Aequilibrierung | | 1 Min. |
| Stop | Zugabe von DCC | |
| Kopplung | | 30 Min. |
| Waschen | DMF | 1 x 1 Min. |
| Waschen | Isopropanol | 2 x 1 Min. |
| Stop | Ninhydrin-Test nach der Kopplung (muss negativ sein) | |

Nach Beendigung der Synthese wurden 0.5 g Decapeptidharz mit Trifluoressigsäure/Wasser (4:1) 2 Stunden geschüttelt. Das Harz wurde abfiltriert, und das Filtrat eingeengt, der Rückstand mit Aether digeriert und das (in Aether unlösliche) Produkt aus Wasser lyophilisiert. Das Produkt wog 125 mg und wies im Massenspektrum (FAB) die erwartete Molmasse 1063 auf.

### Beispiel 9

16 g (0.1 Mol) t-Butyl (2-aminoethyl)carbamat, 17.3 g (0.11 Mol) 5-Chlor-2-pyridincarbonsäure und 1.1 g (0.01 Mol) 1-Hydroxy-2(1H)-pyridon werden in 170 ml Acetonitril bei 20° gerührt. Zum Reaktionsgemisch tropft man eine Lösung aus 22.7 g (0.11 Mol) Dicyclohexylcarbodiimid in 200 ml Acetonitril innerhalb von 30 Minuten. Das Reaktionsgemisch wird über Nacht bei 20° gerührt. Anschliessend wird der Festkörper abgenutscht , und das Filtrat am Wasserstrahlvakuum eingedampft. Der Rückstand wird in 90 ml Methylenchlorid gelöst, und die Lösung langsam mit 120 ml Hexan versetzt, wobei t-Butyl [2-(5-chlor-2-pyridincarboxamido)ethyl]carbamat auskristallisiert. Die Suspension wird bei -10° 90 Minuten gerührt, und das t-Butyl [2-(5-chlor-2-pyridincarboxamido)ethyl]carbamat anschliessend abfiltriert und bei 35° im Vakuumtrockenschrank getrocknet.

### Beispiel 10

16.9 g (0.11 Mol) 3,4-Dihydroxybenzoesäure werden in 170 ml Tetrahydrofuran mit 5 g (0.1 Mol) Hydrazinhydrat und 1.1 g (0.01 Mol) 1-Hydroxy-2(1H)-pyridon versetzt. Zum Reaktionsgemisch gibt man bei 20° innerhalb von 15 Minuten eine Lösung aus 22.7 g (0.11 Mol) Dicyclohexylcarbodiimid in 200 ml Tetrahydrofuran. Das Reaktionsgemisch wird 16 Stunden bei 20° gerührt. Der entstandene Dicyclohexylharnstoff wird abfiltriert, und das Filtrat eingedampft. Der Rückstand wird mit 60 ml Ethanol bei 20° 2 Stunden gerührt, und anschliessend wird das kristalline Rohprodukt abgenutscht und zunächst am Wasserstrahlvakuum und dann am Hochvakuum bei 40° getrocknet, wobei man 3,4-Dihydroxybenzoesäurehydrazid als beiges Pulver erhält.

### Beispiel 11

16.8 g (0.1 Mol) 3,4-Dihydroxybenzoesäurehydrazid, 27.4 g (0.11 Mol) 2-Phthalimidoxyisobuttersäure und 1.1 g (0.01 Mol) N-Hydroxysuccinimid werden in 400 ml Tetrahydrofuran suspendiert, und das Reaktionsgemisch bei 25° innerhalb einer Stunde mit einer Lösung aus 13.9 g (0.11 Mol) Diisopropylcarbodiimid in 200 ml Tetrahydrofuran versetzt. Das Reaktionsgemisch wird 24 Stunden bei 25° gerührt. Der Festkörper wird anschliessend abgenutscht und mit 40 ml Isopropanol und 200 ml t-Butylmethyläther 2 Stunden bei 20° intensiv gerührt. Das Rohprodukt wird abgenutscht und 15 Stunden am Wasserstrahlvakuum bei 40° getrocknet, wobei man 1-(3,4-Dihydroxybenzoyl)-2-[2-methyl-2-(phthalimidoxy)propionyl]hydrazin erhält.

## Patentansprüche

1. Verfahren zur Herstellung von Amiden und Peptiden durch Umsetzen einer Carbonsäure mit einem primären oder sekundären Amin in Gegenwart eines Carbodiimides und einer N-Hydroxyverbindung, dadurch gekennzeichnet, dass die N-Hydroxyverbindung in katalytischer Menge vorliegt und daß N-Hydroxysuccinimid, 1-Hydroxybenzotriazol, 3-Hydroxy-4-oxo-3,4-dihydro-1,2,3-benzotriazin, 1-Hydroxy-2-oxoindolin, 3-Hydroxy-4-oxo-3,4-dihydrochinazolin oder 1-Hydroxy-2(1H)-pyridon, insbesondere N-Hydroxysuccinimid oder 1-Hydroxy-2(1H)-pyridon, als N-Hydroxyverbindung verwendet wird.

2. Verfahren nach Anspruch 1, worin Dicyclohexylcarbodiimid, Diisopropylcarbodiimid, N-Ethyl-N'-(3-dimethylaminopropyl)carbodiimidhydrochlorid oder N-Cyclohexyl-N'-(β-[N-methylmorpholino]ethyl)carbodiimid-p-toluolsulfonat, insbesondere Dicyclohexylcarbodiimid, als Carbodiimid verwendet wird.

3. Verfahren nach Anspruch 1 oder 2, worin eine unsubstituierte oder substituierte aromatisch-aliphatische oder heteroaromatische Carbonsäure oder eine N-acylierte α-Aminosäure, insbesondere (S)-α-[(t-Butylsulfonyl)methyl]hydrozimtsäure, (S)-α-[[[1-(Morpholinocarbonyl)-1-methylethyl]sulfonyl]methyl]hydrozimtsäure, 2-Chinolincarbonsäure, (S)-(t-Butoxycarbonyl)-α-[(S)-α-[(t-butylsulfonyl)methyl]hydrocinnamamido]imidazol-4-propionsäure, (S)-(t-Butoxycarbonyl)-α-[(S)-α-[[[1-(morpholinocarbonyl)-1-methylethyl]sulfonyl]methyl]hydrocinnamamido]imidazol-4-propionsäure, N-(Benzyloxycarbonyl)-L-asparagin, N-(Benzyloxycarbonyl)-L-tyrosin oder N-(2-Chinolylcarbonyl)-L-asparagin, als Carbonsäure verwendet wird.

4. Verfahren nach einem der Ansprüche 1-3, worin eine α-Aminosäure, ein Alkylamin, ein Arylalkylamin oder ein C₃₋₆-Alkylen-disubstituiertes Amin, insbesondere Histidinmethylester, (1S,2R,3S)-3-Amino-4-cyclohexyl-1-cyclopropyl-buten-1,2-diol, 2-(3(S)-Amino-2(R)-hydroxy-4-phenylbutyl)-N-t-butyl-decahydro-(4aS,8aS)-isochinolin-3(S)-carboxamid oder t-Butyl (4-piperidinyloxy)acetat, als primäres oder sekundäres Amin verwendet wird.

5. Verfahren nach einem der Ansprüche 1-4, worin die Umsetzung in einem inerten organischen Lösungsmittel oder Lösungsmittelgemisch, insbesondere in Ethylacetat, Aceton oder Tetrahydrofuran, durchgeführt wird.

6. Verfahren nach einem der Ansprüche 1-5, worin die Umsetzung bei einer Temperatur zwischen 0° und 50°C, vorzugsweise bei Raumtemperatur, durchgeführt wird.

7. Verfahren nach einem der Ansprüche 1-6 zur Herstellung von (S)-α-[(t-Butylsulfonyl)methyl]hydrocinnamamido]imidazolpropionsäuremethylester, dadurch gekennzeichnet, dass als Carbonsäure (S)-α-[(t-Butylsulfonyl)methyl]hydrozimtsäure und als primäres Amin Histidinmethylester verwendet wird.

8. Verfahren nach einem der Ansprüche 1-6 zur Herstellung von N-[(S)-α-[[[1-Methyl-1-(morpholinocarbonyl)ethyl]sulfonyl]methyl]hydro-cinnamoyl]-L-histidinmethylester, dadurch gekennzeichnet, dass als Carbonsäure (S)-α-[[[1-(Morpholinocarbonyl)-1-methylethyl]sulfonyl]methyl]hydrozimtsäure und als primäres Amin Histidinmethylester verwendet wird.

9. Verfahren nach einem der 1-6 zur Herstellung von cis-2-[3(S)-[[N-(Benzyloxycarbonyl)-L-asparaginyl]amino]-2(R)-hydroxy-4-phenylbutyl]-N-t-butyl-decahydro-(4aS,8aS)-isochinolin-3(S)-carboxamid, dadurch gekennzeichnet, dass als Carbonsäure N-(Benzyloxycarbonyl)-L-asparagin und als primäres Amin 2-[3(S)-Amino-2(R)-hydroxy-4-phenylbutyl]-N-t-butyl-decahydro-(4aS,8aS)-isochinolin-3(S)-carboxamid verwendet wird.

## Claims

1. A process for the manufacture of amides and peptides by reacting a carboxylic acid with a primary or secondary amine in the presence of a carbodiimide and a N-hydroxy compound, characterized in that the N-hydroxy compound is present in catalytic amounts and in that N-hydroxysuccinimide, 1-hydroxybenzotriazole, 3-hydroxy-4-oxo-3,4-dihydro-1,2,3-benzotriazine, 1-hydroxy-2-oxoindoline, 3-hydroxy-4-oxo-3,4-dihydroquinazoline or 1-hydroxy-2(1 H)-pyridone, especially N-hydroxysuccinimide or 1-hydroxy-2(1H)-pyridone, is used as the N-hydroxy compound.

2. A process according to claim 1, wherein dicyclohexylcarbodiimide, diisopropylcarbodiimide, N-ethyl-N'-(3-dimethylaminopropyl)carbodiimide hydrochloride or N-cyclohexyl-N'-(β-[N-methylmorpholino]ethyl)carbodiimide p-toluenesulphonate, especially dicyclohexylcarbodiimide, is used as the carbodiimide.

3. A process according to claim 1 or 2, wherein an unsubstituted or substituted aromatic-aliphatic or heteroaromatic carboxylic acid or a N-acylated α-amino acid, especially (S)-α-[(t-butylsulphonyl)methyl]hydrocinnamic acid, (S)-α-[[[1-(morpholinocarbonyl)-1-methylethyl]sulphonyl]methyl]hydrocinnamic acid, 2-quinolinecarboxylic acid, (S)-(t-butoxycarbonyl)α-[(S)-α-[(t-butylsulphonyl)methyl]hydrocinnamamido]imidazole-4-propionic acid, (S)-(t-butoxycarbonyl)-α-[(S)-α-[[[1-(morpholinocarbonyl)-1-methylethyl]sulphonyl]methyl]hydrocinnamamido]imidazole-4-propionic acid, N-(benzyloxycarbonyl)-L-asparagine, N-(benzyloxycarbonyl)-L-tyrosine or N-(2-quinolylcarbonyl)-L-asparagine, is used as the carboxylic acid.

4. A process according to any one of claims 1-3, wherein an α-amino acid, an alkylamine, an arylalkylamine or a C₃₋₆-alkylene-disubstituted amine, especially histidine methyl ester, (1S,2R,3S)-3-amino-4-cyclohexyl-1-cyclopropyl-butene-1,2-diol, 2-(3(S)-amino-2(R)-hydroxy-4-phenylbutyl)-N-t-butyl-decahydro-(4aS,8aS)-isoquinoline-3(S)-carboxamide or t-butyl (4-piperidinyloxy)acetate, is used as the primary or secondary amine.

5. A process according to any one of claims 1-4, wherein the reaction is carried out in an inert organic solvent or solvent mixture, especially in ethyl acetate, acetone or tetrahydrofuran.

6. A process according to any one of claims 1-5, wherein the reaction is carried out at a temperature between 0° and 50°C, preferably at room temperature.

7. A process according to any one of claims 1-6 for the manufacture of (S)-α-[(t-butylsulphonyl)methyl]hydrocinnamamido]imidazole-propionic acid methyl ester, characterized in that (S)-α-[(t-butylsulphonyl)methyl]hydrocinnamic acid is used as the carboxylic acid and histidine methyl ester is used as the primary amine.

8. A process according to any one of claims 1-6 for the manufacture of N-[(S)-α-[[[1-methyl-1-(morpholinocarbonyl)ethyl]sulphonyl]methyl]hydrocinnamoyl]-L-histidine methyl ester, characterized in that (S)-α-[[[1-(morpholinocarbonyl)-1-methylethyl]sulphonyl]methyl]hydrocinnamic acid is used as the carboxylic acid and histidine methyl ester is used as the primary amine.

9. A process according to any one of claims 1-6 for the manufacture of cis-2-[3(S)-[[N-(benzyloxycarbonyl)-L-asparaginyl]amino]-2(R)-hydroxy-4-phenylbutyl]-N-t-butyl-decahydro-(4aS,8aS)-isoquinoline-3(S)-carboxamide, characterized in that N-(benzyloxycarbonyl)-L-asparagine is used as the carboxylic acid and 2-[3(S)-amino-2(R)-hydroxy-4-phenylbutyl]-N-t-butyl-decahydro-(4aS,8aS)-isoquinoline-3(S)-carboxamide is used as the primary amine.

## Revendications

1. Procédé pour préparer des amides et des peptides par réaction d'un acide carboxylique avec une amine primaire ou secondaire en présence d'un carbodiimide et d'un composé N-hydroxylé, caractérisé en ce que le composé N-hydroxylé est présent en une quantité catalytique, et que l'on utilise en tant que composé N-hydroxylé le N-hydroxysuccinimide, le 1-hydroxybenzotriazole, la 3-hydroxy-4-oxo-3,4-dihydro-1,2,3-benzotriazine, la 1-hydroxy-2-oxoindoline, la 3-hydroxy-4-oxo-3,4-dihydroquinazoline ou la 1-hydroxy-2(1H)-pyridone, en particulier le N-hydroxysuccinimide ou la 1-hydroxy-2(1H)-pyridone.

2. Procédé selon la revendication 1, dans lequel on utilise en tant que carbodiimide le dicyclohexylcarbodiimide, le diisopropylcarbodiimide, le chlorhydrate de N-éthyl-N'-(3-diméthylaminopropyl)carbodiimide ou le p-toluènesulfonate de N-cyclohexyl-N'-(β-[N-méthylmorpholino]éthyl)carbodiimide, en particulier le dicyclohexylcarbodiimide.

3. Procédé selon la revendication 1 ou 2, dans lequel on utilise en tant qu'acide carboxylique un acide carboxylique aromatique-aliphatique ou hétéroaromatique, substitué ou non-substitué, ou un acide α-aminé N-acylé, en particulier l'acide (S)-α-[(tert-butylsulfonyl)méthyl]hydrocinnamique, l'acide (S)-α-[[[1-(morpholinocarbonyl)-l-méthyléthyl]sulfonyl]méthyl]hydrocinnamique, l'acide 2-quinoléinecarboxylique, l'acide (S)-(tert-butoxycarbonyl)-α-[(S )-α-[(tert-butylsulfonyl)méthyl]hydrocinnamamido]imidazole-4-propionique, l'acide (S)-(tert-butoxycarbonyl)-α-[(S)-α-[[[1-(morpholinocarbonyl)-1-méthyléthyl]sulfonyl]méthyl]hydrocinnamamido]imidazole-4-propionique, la N-benzyloxycarbonyl)-L-asparagine, la N-(benzyloxycarbonyl)-L-tyrosine ou la N-(2-quinoléylcarbonyl)-L-asparagine.

4. Procédé selon l'une des revendications 1 à 3, dans lequel on utilise en tant qu'amine primaire ou secondaire un acide α-aminé, une alkylamine, une arylalkylamine ou une amine à disubstitution alkylène en C₃₋₆, en particulier l'ester méthylique de l'histidine, le (1S,2R,3S)-3-amine-4-cyclohexyl-1-cyclopropylbutène-1,2-diol, le 2-(3(S)-amino-2(R)-hydroxy-4-phénylbutyl)-N-tert-butyldécahydro-(4aS,8aS )-isoquinoléine-3(S)-carboxamide ou le (4-pipéridinyloxy)acétate de tert-butyle.

5. Procédé selon l'une des revendications 1 à 4, dans lequel la réaction est mise en oeuvre dans un solvant ou dans un mélange de solvants organiques inertes, en particulier dans l'acétate d'éthyle, l'acétone ou le tétra-hydrofuranne.

6. Procédé selon l'une des revendications 1 à 5, dans lequel la réaction est mise en oeuvre à une température de 0 à 50°C, de préférence à la température ambiante.

7. Procédé selon l'une des revendications 1 à 6, pour préparer l'ester méthylique de l'acide (S)-α-[( tert-butyl-sulfonyl)méthyl]hydrocinnamamido]imidazole propionique, caractérisé en ce qu'on utilise en tant qu'acide carboxylique l'acide (S)-α-[(tert-butylsulfonyl)méthyl]hydrocinnamique, et en tant qu'amine primaire l'ester méthylique de l'histidine.

8. Procédé selon l'une des revendications 1 à 6, pour préparer l'ester méthylique de la N-[(S)-α-[[[-1-méthyl-1-(morpholinocarbonyl)éthyl]sulfonyl]méthyl]hydrocinammoyl]-L-histidine, caractérisé en ce qu'on utilise en tant qu'acide carboxylique l'acide (S)-α-[[[1-(morpholinocarbonyl)-l-méthyléthyl]sulfonyl]méthyl]hydrocinnamique, et en tant qu'amine primaire l'ester méthylique de l'histidine.

9. Procédé selon l'une des revendications 1 à 6, pour préparer le cis-2-[3(S)-[[N-(benzyloxycarbonyl)-L-asparaginyl]amino]-2(R)-hydroxy-4-phénylbutyl]-N-tert-butyldécahydro-(4aS,8aS)-isoquinoléine-3(S)-carboxamide, caractérisé en ce qu'on utilise en tant qu'acide carboxylique la N-(benzyloxycarbonyl)-L-asparagine et en tant qu'amine primaire le 2-[3(S)-amino-2(R)-hydroxy-4-phénylbutyl]-N-tert-butyl-décahydro-(4aS,8aS)-isoquinoléine-3(S)-carboxamide.
